# EUROPEAN PATENT SPECIFICATION

(11) **EP 3 090 761 B1**
(45) Date of publication and mention of the grant of the patent: **31.10.2018**
(21) Application number: 16168549.0
(22) Date of filing: 06.05.2016
(51) Int. Cl.: A61L 2/07, A61L 2/26, F22D 1/00

(54) **APPARATUS TO MANAGE THE ENERGY OF HOT STREAMS FROM A STERILIZATION MACHINE, STERILIZATION MACHINE COMPRISING SAID APPARATUS AND CORRESPONDING METHOD TO MANAGE THE ENERGY OF HOT STREAMS AND STERILIZATION METHOD**
GERÄT ZUR VERWALTUNG DER ENERGIE VON HEISSEN STRÖMEN EINER STERILISATIONSMASCHINE, STERILISATIONSMASCHINE DIE DAS GERÄT AUFWEIST UND EIN ENTSPRECHENDES VERFAHREN ZUR VERWALTUNG DER ENERGIE VON HEISSEN STRÖMEN UND STERILISATIONSVERFAHREN
APPAREIL POUR GÉRER L'ÉNERGIE DE CHAUD COURANTS D'UNE MACHINE DE STÉRILISATION, MACHINE STÉRILISATION COMPRENANT LEDIT APPAREIL ET PROCÉDÉ CORRESPONDANT POUR GÉRER L'ÉNERGIE DES COURANTS CHAUD ET MÉTHODE DE STÉRILISATION

(30) Priority: 08.05.2015 IT UD20150064
(43) Date of publication of application: 09.11.2016
(73) Proprietor: Icos Pharma S.p.A., 33080 Zoppola (IT)
(72) Inventor: Zardini, Fabio, 31033 Castelfranco Veneto (TV) (IT); Capovilla, Ivone, 31037 Loria (TV) (IT)
(74) Representative: Petraz, Davide Luigi

(56) References cited:
- DE-A1- 3 144 789
- DE-A1-102013 202 188
- JP-A- 2004 041 529
- US-A- 6 048 502
- US-A1- 2005 189 016

## Description

### FIELD OF THE INVENTION

Embodiments described here concern an apparatus to manage the energy of hot streams from a sterilization machine for objects, such as for example objects used for healthcare, medical or veterinary use, surgical instruments, laboratory instruments or containing devices for laboratory animals or healthcare furnishing elements, for example in hospitals, such as hospital beds or trolleys.

Embodiments also concern a method for the energy management of hot streams from a machine for sterilizing objects.

Other embodiments concern a machine for sterilizing objects comprising said apparatus for the energy management of hot streams.

Still other embodiments concern a method for sterilizing objects that provides an energy management of hot streams from the machine for sterilizing objects.

### BACKGROUND OF THE INVENTION

It is known that objects intended for healthcare, medical or veterinary use must be rendered sterile, such as for example objects used for healthcare, medical or veterinary use, surgical instruments, laboratory instruments or containing devices for laboratory animals or healthcare furnishing elements, for example in hospitals, such as hospital beds or trolleys. It is known that sterilization is carried out in a sterilization chamber at a temperature of about 134-134.5°C, introducing steam at said temperature, generated by a steam generator fed with cold de-mineralized water, usually at around 12-18°C, and the treatment time can be a few minutes.

Sterilization machines are known, which comprise a sterilization chamber, a steam generator that receives cold de-mineralized water and, by means of a heating unit, generates the saturated steam at about 134°C which is supplied to the sterilization chamber.

A first disadvantage is the large amount of energy required to take the cold de-mineralized water to the temperature of about 134°C at which the steam used in the sterilization is generated.

Usually, moreover, the de-mineralized water is produced by an inverse osmosis plant. Such plants, due to their construction and to ensure their correct functioning, need periodic downtimes. If the downtime occurs during a sterilization cycle, it can cause the disadvantage that no more de-mineralized water is available to complete the sterilization cycle in progress.

Furthermore, it must be noted that, between one sterilization cycle and the next, the sterilization chamber can stay inactive even for some hours, but it must in any case be always ready and kept at temperature, to start a new sterilization cycle. To this purpose, the sterilization chamber is provided with a perimeter interspace that is supplied with part of the superheated steam generated, in order to keep the sterilization chamber at temperature and to reduce the times needed to start the sterilization cycle. Otherwise, if the chamber were left to cool between one cycle and the next, it would be necessary, with every new cycle, to heat the whole machine again which, considering the considerable mass of the construction materials in play, above all steel, would represent an enormous waste of energy, cost and time. On the contrary, in this way the steam in the interspace keeps the sterilization chamber ready and at temperature.

Moreover, for the purposes of sterilization, the superheated steam generated is also introduced directly into the sterilization chamber. At end-of-cycle, the used steam is extracted from the chamber. At the end of the working day, moreover, the steam is also extracted from the interspace. The used steam extracted is usually at a temperature slightly below the 134°C required for sterilization, for example about 125-130°C and, by means of condensation dischargers, is transformed into condensation, which is discharged as a stream of hot water to the floor, generally at a temperature a little below 100°C, usually hot water at about 98°C. One disadvantage, therefore, is that hot streams are discharged, essentially water, at a temperature a little below 100°C, hence dispersing all the enthalpy content that they have. Moreover, drains must be made that are suitable to receive these hot streams, of the closed or forced type, sized correctly and made of materials, usually steel or cast iron, that resist the high temperatures in question.

Usually, before the sterilization cycle is started, standard tests are carried out on the temperature and penetration capacity of the superheated steam, to verify that the machine is in operating conditions within a predetermined range of values so that, if successfully passed, the sterilization cycle can be started (validation of the sterilization cycle of the machine).

It is also known that, in order to pass the preliminary tests as above, it is necessary to inject saturated steam, clean and without any residual air. To this purpose, before the start of the sterilization cycle, an operation to extract the air present in the treatment chamber is repeatedly carried out, normally three times. This is done by a suction device, such as for example a liquid-ring vacuum pump or a Venturi effect pump (also called water pump), but this normally requires large quantities of cold water in order to function. For example, in the case of a vacuum pump, initially it is driven to put the chamber under vacuum and to extract a large part of the air/oxygen present in the chamber. Then, still operating in a vacuum, superheated steam is introduced into the chamber as well and, with the two subsequent removal operations, the vacuum pump extracts all the residual air together with the steam, which is normally damp steam at a temperature in the order of about 130°C. Normally, for the functioning of the vacuum pump with liquid ring, a feed of cold water is provided, at about 12-18°C, which can also be a feed of 5 1/min. The cold water serves to cool the liquid ring of the vacuum pump and is mixed with the hot streams extracted as described above and discharged. Therefore, ultimately, the hot streams of steam and air extracted in these operations, and mixed with cold water, are discharged to the floor, with the same disadvantages of waste of energy of the enthalpy content of said streams, and also the need to construct special drains for the hot streams. Furthermore, there is a waste of the volume of cold water that is introduced into the liquid-ring vacuum pump for its functioning. As we said, there is a similar waste of water if a Venturi effect pump is used.

At the end of these vacuum removal operations, the chamber, from which the air has been suitably extracted as required by the sterilization protocol, can be put to temperature by introducing superheated steam and subjected to the preliminary tests described above.

Also at the end of the sterilization cycle, the residual steam in the chamber is extracted by the suction device and, in this case too, the hot streams extracted are discharged to the floor, with all the disadvantages described above.

Another disadvantage connected to the vacuum removal of said hot streams, generally at a temperature in the order of about 130°C, from the sterilization chamber, is that there may be an accumulation of lime scale in the suction device, with a consequent deterioration of the operating performance. Furthermore, at these temperatures, the suction device can be very stressed thermally, which can reduce its working life.

Moreover, in the case of a battery of sterilization machines of the type in question, disposed in series, each of which discharges the hot streams to the floor, it may be that not all the machines have their own dedicated drain for the hot streams and instead share a single line to discharge them to the floor.

One disadvantage of this situation is that these hot streams, discharged in a single discharge line, can create counter-pressure or super-pressure effects backward from the sterilization machines further downstream toward those more upstream with respect to the floor discharge line. One disadvantage of this super-pressure is that it can prevent the completion of the preliminary functioning tests, and hence of the sterilization cycle, since it can prevent the machine upstream from reaching the correct sterilization temperature. In substance therefore, if there is a single drain of the hot streams shared by a battery of sterilization machines, the sterilization machines upstream do not reach the correct temperature and in practice become unusable because they do not pass the preliminary validation tests. This can be obviated with civil construction works to build dedicated drains for each of the machines, but this obviously causes higher costs and longer times.

Document DE-A-3144789 describes a method and an apparatus to recover heat in autoclaves heated directly by steam.

Document JP-A-2004041529 describes a system to recover steam used in a sterilization apparatus.

Document US-A-6,048,502 describes a sterilization mechanism with recirculating water.

Document US-A-2005/0189016 describes a recirculation system to receive and recirculate an effluent arriving from a sterilization apparatus.

There is therefore a need to perfect an apparatus to manage the energy of hot streams from a sterilization machine for objects, a sterilization machine for objects comprising said apparatus, a corresponding method to manage the energy of hot streams and a sterilization method that can overcome at least one of the disadvantages of the state of the art. In particular, one purpose of the present invention is to recover the enthalpy content of the hot streams exiting from the sterilization machine, which would otherwise be wasted.

Another purpose of the present invention is to obtain stream discharges that have much lower temperatures than the hot streams described above, so as to simplify and make more economical the civil constructions built to receive said discharges. This is also to prevent the accumulation of lime scale and a drop in the performance of the suction device and allow to extend its working life.

Another purpose is to prevent problems in reaching the operative sterilization temperature in the sterilization machines in question.

Another purpose is to supply a reserve of de-mineralized water ready for use if the inverse osmosis plant is temporarily stopped.

The Applicant has devised, tested and embodied the present invention to overcome the shortcomings of the state of the art and to obtain these and other purposes and advantages.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

### SUMMARY OF THE INVENTION

The present invention is set forth and characterized in the independent claims, while the dependent claims describe other characteristics of the invention or variants to the main inventive idea.

In accordance with some embodiments, an apparatus is provided to manage the energy of hot streams of a sterilization machine. According to one embodiment the apparatus comprises:
- a heat exchange chamber;
- a heat exchanger;
- a water collection chamber;
wherein the heat exchange chamber is configured to be at least connected at entrance to an inlet pipe for hot streams arriving from a sterilization chamber of the sterilization machine, a heat exchange element being provided inserted into the heat exchange chamber and connected to the hot streams inlet pipe,
wherein the heat exchanger is configured to be connected at entrance to a cooling water inlet pipe and to a vacuum extraction branch of a mixed hot stream connected to the sterilization chamber and at exit to a cooling water introduction pipe connected to the water collection chamber and to an outlet pipe for the cooled streams connected to a suction device, wherein the water collection chamber is connected to the heat exchange chamber by means of a transfer pipe and is configured to be connected at entrance to said cooling water introduction pipe and wherein said water collection chamber is also connected in use to a suction device at entrance via a vacuum circuit water inlet pipe arriving from an exit of the suction device to supply water from the suction device to the water collection chamber and at exit via a condensation water outlet pipe connected at entrance to the suction device to supply condensation water from said water collection chamber to said suction device.

In accordance with other embodiments, a container is provided to manage the energy of hot streams of a sterilization machine. According to one embodiment, the container includes:
- a heat exchange chamber and a water collection chamber disposed above or below the heat exchange chamber;
- at least one heat exchange element provided in the heat exchange chamber;
- a separation wall between the heat exchange chamber and the water collection chamber;
- a transfer pipe between the heat exchange chamber and the water collection chamber, passing through the separation wall;
wherein the heat exchange chamber is provided at least with an entrance, associated with said heat exchange element, connecting a hot streams inlet pipe arriving from a sterilization chamber of the sterilization machine;
wherein the water collection chamber is provided at least with a connection entrance of a cooling water introduction pipe and is connected in use to a suction device via a connection entrance of a vacuum circuit water inlet pipe arriving from an exit of the suction device to supply water from the suction device to the water collection chamber and via a connection exit of a condensation water outlet pipe connected at entrance to said suction device to supply condensation water from said water collection chamber to said suction device.

In accordance with other embodiments, a method is provided to manage the energy of hot streams of a sterilization machine. According to one embodiment, the method comprises:
- cooling, in a heat exchange chamber, hot streams arriving from a sterilization chamber of the sterilization machine;
- performing a heat exchange, by means of a heat exchanger, between cold water and a mixed hot stream arriving from the sterilization chamber, to cool the mixed hot stream;
- introducing the cold water subjected to heat exchange in the heat exchanger into a water collection chamber and introducing the cooled mixed hot stream into a suction device;
- transferring into the water collection chamber the exhausted hot streams cooled in the heat exchange chamber;
- introducing into the suction device the water contained in the water collection chamber, mixing it with the cooled mixed hot stream arriving from the heat exchanger;
- transferring into the water collection chamber the mixed hot stream cooled and mixed with the water contained in the water collection chamber at exit from the suction device;
- discharging freely into a water discharge line an excess liquid present in the heat exchange chamber and the excess water in the water collection chamber.

In accordance with other embodiments, a machine for sterilizing objects is provided. According to one embodiment, the machine comprises:
- a sterilization chamber,
- a steam generator,
- a suction device,
- an apparatus to manage the energy of hot streams that comprises:
   - a heat exchange chamber;
   - a heat exchanger;
   - a water collection chamber;
wherein the heat exchange chamber is at least connected at entrance to a hot streams inlet pipe arriving from the sterilization chamber,
wherein the heat exchanger is connected at entrance to a cooling water inlet pipe and to a vacuum extraction branch of mixed hot stream connected to the sterilization chamber and at exit to a cooling water introduction pipe connected to the water collection chamber and to an outlet pipe for the cooled streams connected to the suction device, and wherein the water collection chamber is connected to the heat exchange chamber by means of a transfer pipe and is connected at entrance to said cooling water introduction pipe and also to a vacuum circuit water inlet pipe arriving from an exit of the suction device and at exit to a condensation water outlet pipe connected at entrance to the suction device.

In accordance with still other embodiments, a method for sterilizing objects is provided. According to one embodiment the method comprises:
- putting under vacuum once or several times a sterilization chamber of a sterilization machine to extract a mixed hot stream, by means of a suction device;
- producing and introducing steam into the sterilization chamber to perform a sterilization operation, extracting steam from the sterilization chamber that is condensed, producing hot streams;
- drying the sterilized objects, putting under vacuum once or several times the sterilization chamber extracting another mixed hot stream, by means of the suction device;
wherein said method also provides to:
- cool, in a heat exchange chamber, said hot streams arriving from the sterilization chamber;
- perform a heat exchange, by means of a heat exchanger, between cold water and said mixed hot stream arriving from the sterilization chamber, to cool the mixed hot stream;
- introducing the cold water subjected to heat exchange in the heat exchanger into a water collection chamber and introducing the cooled mixed hot stream into a suction device;
- transferring into the water collection chamber the exhausted hot streams cooled in the heat exchange chamber;
- introducing into the suction device the water contained in the water collection chamber, mixing it with the cooled mixed hot stream arriving from the heat exchanger;
- transferring into the water collection chamber the mixed hot stream cooled and mixed with the water contained in the water collection chamber at exit from the suction device;
- discharging freely into a water discharge line an excess liquid present in the heat exchange chamber and the excess water in the water collection chamber.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example, with reference to the attached drawings wherein:
- fig. 1 is a schematic representation of a sterilization machine that comprises an apparatus to manage the energy of hot streams in accordance with embodiments described here;
- fig. 2 is a schematic representation of a sterilization machine that comprises an apparatus to manage the energy of hot streams in accordance with other embodiments described here;

- fig. 3 is a schematic representation of an apparatus to manage the energy of hot streams for a sterilization machine in accordance with embodiments described here;
- fig. 4 is another schematic representation of an apparatus to manage the energy of hot streams for a sterilization machine in accordance with embodiments described here;
- fig. 5 is a schematic representation of an apparatus to manage the energy of hot streams for a sterilization machine in accordance with other embodiments described here;
- fig. 6 is another schematic representation of an apparatus to manage the energy of hot streams for a sterilization machine in accordance with embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawing. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described here concern an apparatus 10 to manage the energy of hot streams for a sterilization machine 50.

Embodiments described here also concern a sterilization machine 50 comprising an apparatus 10 as described here.

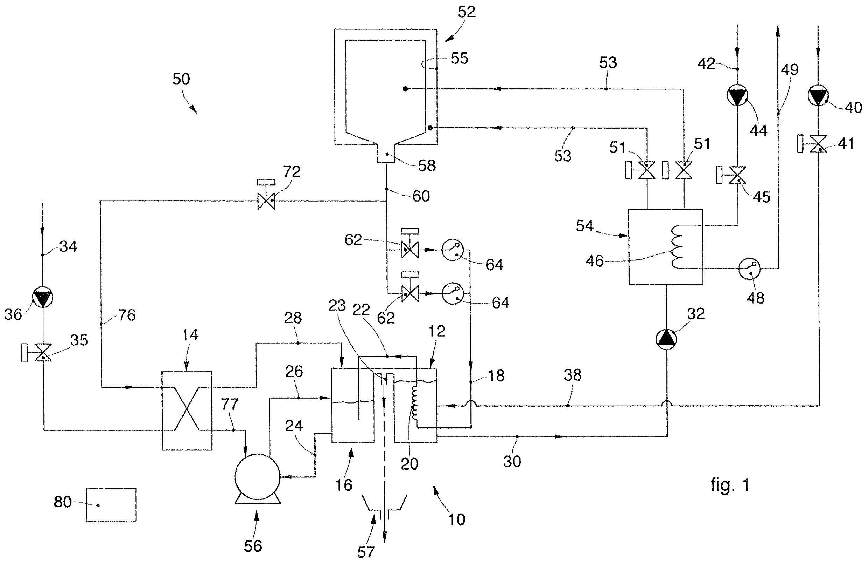

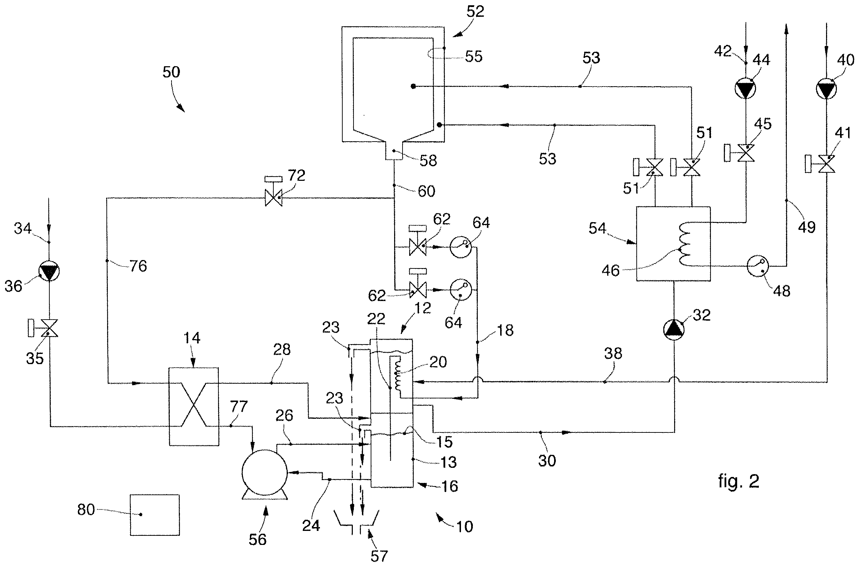

Figs. 1 and 2 are used to describe embodiments, combinable with all the embodiments described here, of a sterilization machine 50 that includes:
- a sterilization chamber 52,
- a steam generator 54,
- a suction device 56 able to generate a vacuum,
- an apparatus 10 to manage the energy of hot streams.

Figs. 1, 2, 3 and 4 are used to describe possible embodiments, combinable with all the embodiments described here, of the apparatus 10 which includes:
- a heat exchange chamber 12;
- a heat exchanger 14;
- a water collection chamber 16.

According to some embodiments, the heat exchange chamber 12 is configured to be at least connected at entrance to an inlet pipe for the hot streams 18 arriving from the sterilization chamber 52 of the sterilization machine 50.

In possible embodiments, the apparatus 10 is also configured to carry out a pre-heating of de-mineralized water to be supplied to the steam generator 54.

According to these embodiments, the heat exchange chamber 12 is able to be connected both to a source of de-mineralized water, and to the steam generator 54 and also to the sterilization chamber 52, from which it is able to receive hot streams to pre-heat the de-mineralized water to be supplied to the steam generator 54.

Here, the expression "hot streams" means condensations to a temperature a little below 100°C, for example between 97°C and 99°C, or streams of steam, mixed streams of steam and air, hot air or similar hot streams.

Moreover, according to some embodiments, the heat exchange chamber 12 is also connected to the water collection chamber 16, to transfer to it the used hot streams cooled in the heat exchange chamber 12, for example by means of a transfer pipe 22 as will be explained in more detail hereafter. The used hot streams cooled arriving from the heat exchange chamber 12 can be hot streams that have been used in the heat exchange chamber 12 for example to pre-heat de-mineralized water contained therein. In these embodiments, therefore, the heat exchange chamber 12 can be a chamber for pre-heating the de-mineralized water.

Furthermore, the water collection chamber 16 is able to be connected to the suction device 56, to circulate the water in the vacuum circuit, and also to the heat exchanger 14, to receive water subjected to heat exchange with hot streams arriving from the sterilization chamber 52.

According to some embodiments, described using figs. 1, 2, 3 and 4, and combinable with all the embodiments described here, a hot streams inlet pipe 18 can be provided, associated with the heat exchange chamber 12. The hot streams inlet pipe 18 can be configured to receive hot streams, for example typically condensed saturated steam at about 98°C, from the sterilization chamber 52, continuously or discontinuously, during the sterilization cycle. The sterilization chamber 52, traditionally, can be provided with a temperature maintenance interspace 55, fed with steam. The hot streams inlet pipe 18 can be for example a single collector, to which various streams of condensed steam converge, arriving from the sterilization chamber 52.

The sterilization chamber 52 in particular can be provided with a steam discharge collector 58, to which all the discharges converge, both steam or air mixed with steam, arriving from the sterilization chamber 52, i.e. from inside it, or, at the end of the working day, from the temperature maintenance interspace 55 and possibly from other common auxiliary steam services in the sterilization machines in question and not described in detail here. The steam discharge collector 58 can be connected to a steam discharge pipe 60, in turn connected to one or more control valves 62 connected in line with respectively one or more condensation dischargers 64. The number of condensation dischargers 64 can be correlated to the number of steam discharges provided, for example directly from inside the sterilization chamber 52, from the temperature maintenance interspace 55 at the end of the working day and from possible other auxiliary steam services provided. A condensation discharger 64 as used in the embodiments described here is configured to transform the steam arriving from the sterilization chamber 52 into a hot stream of condensation, for example normally at around 98°C. The one or more condensation dischargers 64 are in turn connected to the hot streams inlet pipe 18. To perform sterilization at the required temperature, the steam introduced into the sterilization chamber 52 must be extracted frequently during the sterilization operation, according to the temperature in the sterilization chamber 52, to prevent it from depositing in the sterilization chamber 52 and therefore from not performing its sterilization function.

The one or more condensation dischargers 64 are therefore used to discharge the steam as condensation when necessary during the sterilization cycle, using a valve with a temperature sensor associated therewith, for example of the bimetallic foil type or float, which, if it feels a temperature lower than a threshold value set as a function of the sterilization operation, opens the steam exit circuit and the steam is condensed. These hot streams of condensation are those supplied by the hot streams inlet pipe 18 to the heat exchange chamber 12.

In some embodiments, a heat exchange element 20 can also be provided, inserted into the heat exchange chamber 12 and connected to the hot streams inlet pipe 18. The heat exchange element 20 allows to cool the condensations arriving from the hot streams inlet pipe 18. In this way, it is possible to discharge water at a lower temperature than in the state of the art.

In embodiments described using figs. 1, 2, 3 and 4, the heat given up by cooling the condensations arriving from the hot streams inlet pipe 18 can advantageously be used to pre-heat de-mineralized water in view of the generation of steam in the steam generator 54. In particular, the heat exchange element 20 can be configured to pre-heat de-mineralized water contained in the heat exchange chamber 12, exploiting the heat contribution of the hot streams supplied by the hot streams inlet pipe 18. By means of this pre-heating, the condensations arriving from the hot streams inlet pipe 18 are cooled, since they give up a large part of their heat energy to the de-mineralized water, pre-heating it. This heat contribution can be considerable, since they are often condensations of saturated steam at about 98°C which would otherwise be discharged and wasted. Advantageously, the de-mineralized water can be pre-heated in the heat exchange chamber 12 by the heat exchange element 20 fed by the hot streams inlet pipe 18 at a temperature in the order of 60°C-70°C, for example 65°C.

In possible implementations, the heat exchange element 20 can be a heating element, such as a heating coil connected to the hot streams inlet pipe 18 and able to be immersed in the de-mineralized water contained in the heat exchange chamber 12. In this way, the condensations of saturated steam pass through the heating coil, pre-heating the de-mineralized water.

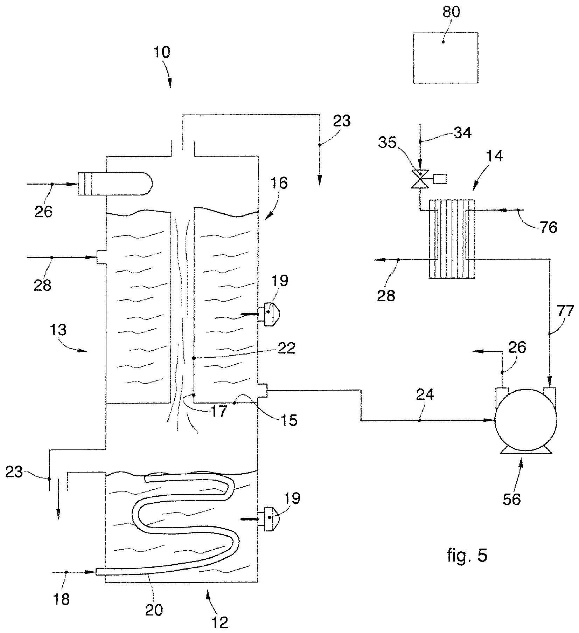

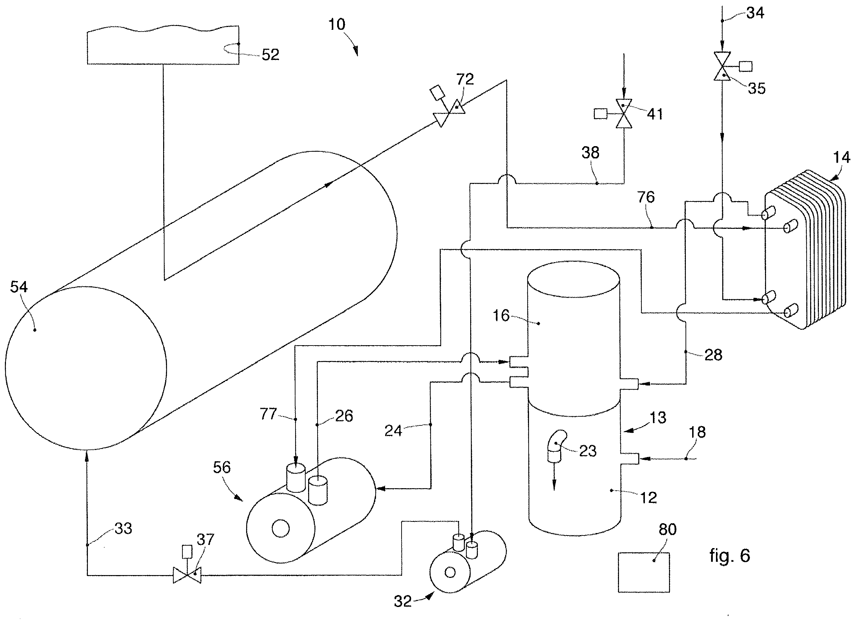

In other embodiments, described using figs. 5 and 6, the condensations arriving from the hot streams inlet pipe 18 are cooled, without recovering the heat given up by them and in this case the heat exchange element 20 is configured to obtain the desired cooling.

In embodiments described here, a pipe 38 to supply de-mineralized water can also be provided.

In embodiments described using figs. 1, 2, 3 and 4, the de-mineralized water supply pipe 38 can be connected at entrance to the heat exchange chamber 12 and able to supply de-mineralized water to the heat exchange chamber 12.

In some embodiments, combinable with all the embodiments described here, the de-mineralized water supply pipe 38 can be associated with a de-mineralized water introduction pump 40, which is configured to take de-mineralized water from a source of de-mineralized water. One example of a source of de-mineralized water can be an inverse osmosis plant for the production of de-mineralized water. Usually the de-mineralized water supplied is cold, about 12°C-18°C, for example about 15°C.

Furthermore, in some embodiments, combinable with all the embodiments described here, the de-mineralized water supply pipe 38 can be associated with an electro valve 41. The electro valve 41 can be disposed in line, downstream of the de-mineralized water introduction pump 40.

In some embodiments, described using figs. 1, 2, 3 and 4, a de-mineralized water exit pipe 30 can also be provided, connected at exit to the heat exchange chamber 12 and able to supply pre-heated de-mineralized water to the steam generator 54. The de-mineralized water exit pipe 30 can be associated with a de-mineralized water introduction pump 32, which is configured to pump de-mineralized water to the steam generator 54.

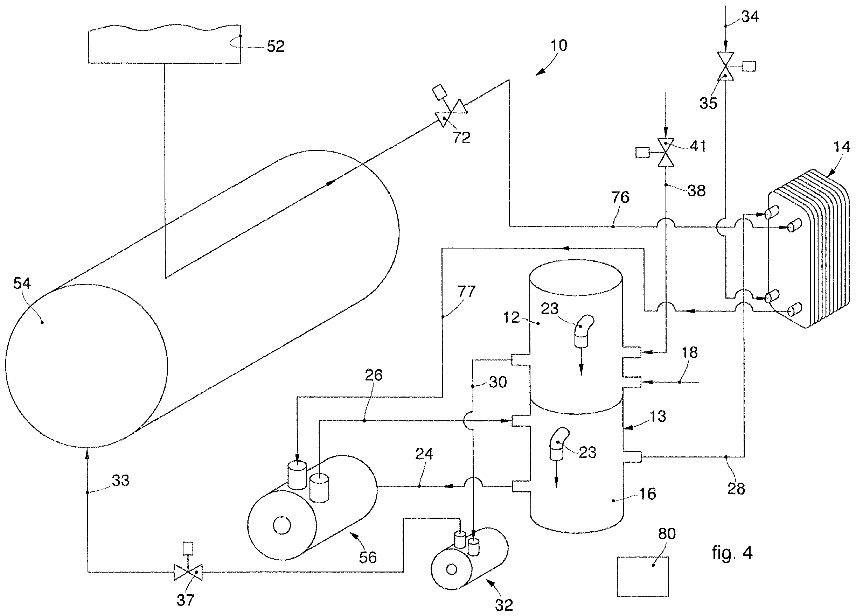

In some embodiments, combinable with all the embodiments described here, downstream of the de-mineralized water introduction pump 32 an entrance pipe 33 to the steam generator 54 can be provided, possibly associated with an element to intercept/adjust the liquid stream, such as an electro valve 37 (see figs. 4 and 6).

In embodiments described using figs. 5 and 6, the de-mineralized water supply pipe 38 can be connected, on the contrary, directly to the de-mineralized water introduction pump 32, and not to the heat exchange chamber 12 and, in this case the de-mineralized water exit pipe 30 is not provided.

The heat exchange chamber 12 can be provided with a level-switch device 21, configured to provide a signal of the level of the de-mineralized water. For example, the level-switch can be the float type. In this way, by means of the level-switch device 21, it is possible to control the level of de-mineralized water inside the heat exchange chamber 12, in order for example to keep the minimum filling level, and also to signal if a maximum level has been exceeded.

In some embodiments, combinable with all the embodiments described here, the steam generator 54 can be typically associated with a heating element 46 which is able to heat the de-mineralized water accumulated therein, to generate the steam at the conditions of temperature and saturation necessary for sterilization. Generally, the temperature required for sterilization is about 134°C-134.5°C. An example of a heating element 46 is an electric resistance. Another example of a heating element 46 is a heating coil inside which steam is able to flow, arriving from a heating steam introduction pipe 42 associated with a heating steam introduction pump 44 and a steam control electro valve 45. After it has been used, the steam that flows in the heating element 46 formed by a heating coil is supplied to a condensation discharger 48 and from here the condensation is extracted using a condensation exit pipe 49. The steam generated by the steam generator 54 that vaporizes the de-mineralized water is sent to the sterilization chamber 52 by one or more steam introduction pipes 53 associated with respective steam control valves 51. For example, embodiments described using figs. 1 and 2, and combinable with all the embodiments described here, can provide a steam introduction pipe 53 to introduce steam inside the sterilization chamber 52 and another steam introduction pipe 53 to introduce steam inside the temperature maintenance interspace 55.

Moreover, in some embodiments, combinable with all the embodiments described here, the apparatus 10 can be provided with a transfer pipe 22, by means of which to transfer liquid from the heat exchange chamber 12 to the water collection chamber 16 (see figs. 1, 2, 3 and 4 for example), or from the water collection chamber 16 to the heat exchange chamber 12 (see figs. 5 and 6 for example).

For example, with reference to the embodiments described using figs. 1, 2, 3 and 4, the transfer pipe 22 can be connected to the heat exchange element 20 and can be able to introduce into the water collection chamber 16 the cooled hot streams, i.e., whose temperature has been lowered because they have been subjected to heat exchange in the heat exchange chamber 12 to pre-heat the de-mineralized water intended for the steam generator 54.

In some embodiments, combinable with all the embodiments described here, both the heat exchange chamber 12 and the water collection chamber 16 can be provided with overflow vent or discharge elements 23, which can be in common (see fig. 1 for example), or separate (see figs. 2, 3, 4, 5 and 6 for example), to discharge freely to a suitable water discharge line 57. This is for example to prevent excessive accumulations of liquids.

Advantageously, thanks to the fact that the condensations arriving from the hot streams inlet pipe 18 are cooled in the heat exchange chamber 12, the water discharged is at a much lower temperature than in the state of the art, no longer around 98°C, but at a temperature of around 30°C-60°C, in particular, 40°C-60°C, for example if it comes from the water collection chamber 16, or in the order of 60°C-70°C, in particular 50°C-60°C, if it comes from the heat exchange chamber 12.

In some embodiments, combinable with all the embodiments described here, a condensation water outlet pipe 24 can also be provided, connected at exit to the water collection chamber 16. The condensation water outlet pipe 24 is able to supply the suction device 56 with the water contained in the water collection chamber 16.

Furthermore, in some embodiments, combinable with all the embodiments described here, a vacuum circuit water inlet pipe 26 can also be provided, connected at entrance to the water collection chamber 16. The vacuum circuit water inlet pipe 26 is able to supply the water collection chamber 16 with the water arriving from the suction device 56.

Furthermore, in some embodiments, combinable with all the embodiments described here, a cooling water introduction pipe 28 can be provided, connected at entrance to the water collection chamber 16. The cooling water introduction pipe 28 is able to supply the water collection chamber 16 with the pre-heated water arriving from the heat exchanger 14.

In some embodiments, combinable with all the embodiments described here, a cooling water introduction pipe 34 can be provided, connected to the heat exchanger 14. The cooling water introduction pipe 34 is able to supply cold water to the heat exchanger 14. The cold water is intended for heat exchange with a hot stream arriving from the sterilization chamber 52, as will be described in more detail hereafter. The cold water can be drinking water for example, or water supplied from the water mains or aqueduct, and can be at a temperature between 12°C and 18°C, for example about 15°C. The cooling water introduction pipe 34 can be associated with a cooling water introduction pump 36, which is configured to pump the cold water to the heat exchanger 14. Furthermore, the cooling water introduction pipe 34 can be associated with an electro valve 35. The electro valve 35 can be disposed in line, downstream of the cooling water introduction pump 36.

In some embodiments, combinable with all the embodiments described here, a vacuum extraction branch of a mixed hot stream 76 can also be provided, which branches off from the steam discharge pipe 60 and is associated with a steam control electro valve 72. The vacuum extraction branch of a mixed hot stream 76 is connected at entrance to the heat exchanger 14, to exchange heat with the cold water arriving from the cooling water introduction pipe 34. A vacuum outlet pipe for the cooled streams 77 is provided, at exit from the heat exchanger 14 and connected to the vacuum extraction branch of a mixed hot stream 76. Inside the vacuum outlet pipe for the cooled streams 77 a stream is able to flow which, arriving from the vacuum extraction branch of a mixed hot stream 76, is cooled in the heat exchanger 14 and supplied to the suction device 56.

In some embodiments, combinable with all the embodiments described here, the vacuum extraction branch of a mixed hot stream 76 can typically be used to extract a mixed hot stream which can be formed of air and/or steam mixed with air and/or steam, arriving from the sterilization chamber 52 in the stages prior to the start of the sterilization cycle, or in the final stage of the sterilization cycle, where drying is carried out. The stream of air and/or steam mixed with air is cooled in the heat exchanger 14 by heat exchange with cold water. In this way, the suction device 56 receives from the vacuum outlet pipe for the cooled streams 77 a stream at a reduced temperature compared with what happens normally and therefore does not cause accumulations of lime scale and drop in the performance of the suction device, and does not subject the suction device 56 to excessive heat stresses, thus promoting an extended working life, since it no longer operates at around the 130°C of the damp steam extracted from the sterilization chamber, but at a temperature for example in the order of about 40°C-60°C, in particular about 45°C-55°C.

In possible embodiments, combinable with all the embodiments described here, the heat exchanger 14 can be a plate-type heat exchanger, or a heat exchanger with concentric tubes.

One example of a suction device 56 usable in association with the embodiments described here can be a vacuum pump, in particular a liquid-ring vacuum pump.

Furthermore, we must underline here that a vacuum pump usable in association with the embodiments described here can essentially be understood as a mechanical device used to create and maintain a vacuum, i.e. a condition of pressure lower than atmospheric pressure, extracting the gas contained in the vacuum chamber to which the device is connected through conduits. The liquid-ring vacuum pump is a vacuum pump used to move liquids or gases, which is normally formed by a cylindrical compartment inside which a rotor with radial blades rotates, with a rotation motion that ensures the formation of a liquid layer in correspondence with the wall of the compartment. The rotor is located in a non-central position and therefore chambers are formed, delimited by the blades of the rotor, with a variable volume, in which the fluid passes and is compressed. Typically they are used to pump large quantities of steam without using an intermediate condenser. In this type of pump, the water is supplied continuously, both to renew the liquid ring and also for cooling, and causes the steam pumped to condense, with a capacity that depends on the sizes of the pump and on the functioning temperature.

Another example of a suction device 56 usable in association with the embodiments described here can be a Venturi effect pump (also called water pump). These pumps exploit the Venturi effect of a current of liquid in the presence of a narrow portion where there is a reduction in pressure and an increase in speed, and they are used to create conditions of depression or vacuum in a desired space. Advantageously, the Venturi effect pump can be equipped with a tank or container to recover the water used for its functioning, so that no water is wasted.

In possible embodiments, combinable with all the embodiments described here, a control unit or system controller 80 can be provided, which can comprise a central processing unit or CPU, an electronic memory, possibly an electronic database and auxiliary circuits (or I/O). A program (or computer instructions) readable by the control unit 80 can determine which tasks can be done in accordance with the energy management method connected to the apparatus 10 and the sterilization method connected to the machine 50, according to the present description. In some embodiments, the program is a software readable by the control unit 80. The control unit 80 includes a code to generate and memorize information and data introduced or generated during the course of the method according to the present description.

For example, the control unit 80 can control the functioning of the apparatus 10 and the machine 50 so as to perform the operations and steps described above, for example controlling in a coordinated manner the functioning of the electro valves 35, 41, 45, 51, 62, of the pumps 32, 36, 40, 44, of the condensation dischargers 48, 64, of the suction device 56 and of the steam generator 54.

Fig. 1 is used to describe embodiments, combinable with all the embodiments described here, of the apparatus 10 and the machine 50 in which the heat exchange chamber 12 and the water collection chamber 16 are two separate and distinct containers.

Figs. 2, 3, 4, 5 and 6 are used to describe embodiments, combinable with all the embodiments described here, of the apparatus 10 and the machine 50 in which the heat exchange chamber 12 and the water collection chamber 16 are formed by a single container 13, which is provided with an intermediate separation wall 15. The container 13 can be a cylindrical tank, for example. The separation wall 15 divides the internal space of the container 13, delimiting the heat exchange chamber 12 and the water collection chamber 16.

In possible implementations, the transfer pipe 22 can pass through the separation wall 15. For example, the separation wall 15 can have an aperture 17 for the passage of the transfer pipe 22.

For example, with reference to embodiments described using figs. 2, 3 and 4 and combinable with all the embodiments described here, the heat exchange chamber 12 can be disposed above the water collection chamber 16. With reference to embodiments described using figs. 5 and 6 and combinable with all the embodiments described here, the heat exchange chamber 12 can instead be disposed below the water collection chamber 16.

In possible implementations, for example with reference to figs. 2, 3 and 4, the aperture 17 can be suitably provided with washers, to seal the heat exchange chamber 12 and the water collection chamber 16 hydraulically.

The embodiments in which the heat exchange chamber 12 and the water collection chamber 16 are formed by a single container 13 can be advantageous since they allow to increase the compactness of the apparatus 10.

In the embodiments described using figs. 2, 3, 4, 5 and 6, the heat exchange chamber 12 and the water collection chamber 16 can each be provided with their own overflow vent or discharge element 23. The overflow vent or discharge elements 23 can be disposed in cooperation with the water discharge line 57.

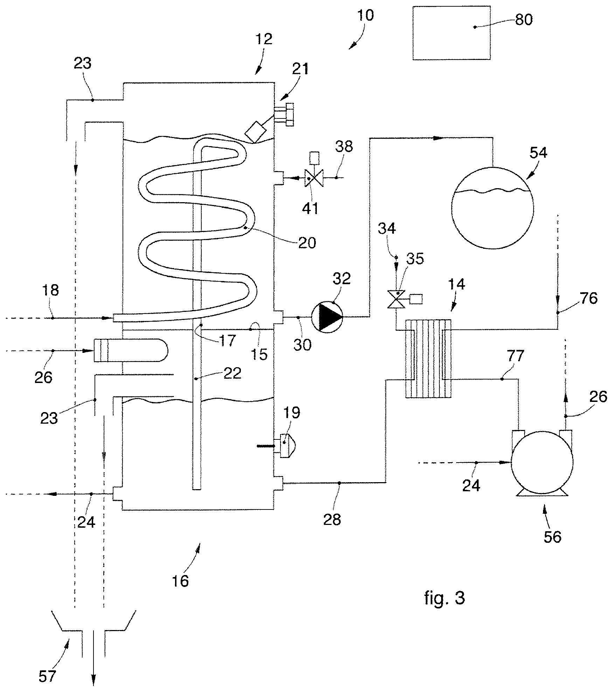

Fig. 3 is used to describe embodiments, combinable with all the embodiments described here, of the apparatus 10 in which a thermostat device 19 is provided, associated with the water collection chamber 16 and the level-switch device 21 associated with the heat exchange chamber 12.

As described above, in some embodiments the machine 50 mainly provides to use two streams of water, i.e. a stream of cold water, for the functioning of the suction device 56, supplied by the cooling water introduction pipe 34, and a stream of cold de-mineralized water to generate steam, supplied by the de-mineralized water supply pipe 38.

The stream of cold water, provided for the functioning of the suction device 56, is also advantageously used in the heat exchanger 14 to cool the damp steam and the air extracted from the sterilization chamber arriving from the vacuum extraction branch of a mixed hot stream 76. Consequently, the stream of cold water, after the heat exchange, is heated to about 50°C-60°C and is introduced into the water collection chamber 16 where the accumulated water is thermostated and stabilized at about 30°C-40°C, for example using the thermostat device 19.

The water accumulated in the water collection chamber 16 is used to supply and cool the suction device 56, by means of the condensation water outlet pipe 24. Therefore, the water supplied by the condensation water outlet pipe 24 is in practice used both to cool the steam mixed with air of the vacuum extraction branch of a mixed hot stream 76, and also to supply water for the functioning of the suction device 56.

Since in the embodiments described using figs. 1, 2, 3 and 4, the transfer pipe 22 transfers the hot streams of condensation used by the heat exchange element 20 to the water collection chamber 16, if the temperature of the water accumulated therein, advantageously monitored by the thermostat device 19, increases excessively, for example more than said 30°C-40°C, more cold water is commanded to be introduced by means of the cooling water introduction pipe 28.

On the contrary, the damp steam mixed with air arriving, before the start of the sterilization cycle (validation stage), from the sterilization chamber 52 by means of the vacuum extraction branch of a mixed hot stream 76, is cooled by the stream of cold water supplied by the cooling water introduction pipe 34 at a lower temperature, about 50°C, thanks to the heat exchanger 14. This cooled stream is introduced into the suction device 56, is mixed here with the water arriving from the condensation water outlet pipe 24 and is subsequently expelled, by means of the vacuum circuit water inlet pipe 26, into the water collection chamber 16. Advantageously, the vacuum circuit water inlet pipe 26 is configured to introduce into the water collection chamber 16 the stream of water arriving from the suction device 56 tangentially to the internal walls of the water collection chamber 16. In this way, the air creates a vortex effect which makes the condensation deposit on the surface and bottom of the water collection chamber 16, while the air is expelled by the overflow vent or discharge element 23.

With regard to the stream of cold de-mineralized water to generate steam, in embodiments described using figs. 1, 2, 3 and 4, it can be introduced into the heat exchange chamber 12 by the de-mineralized water supply pipe 38, and here it is heated by the enthalpy contribution of the hot streams of condensation arriving from the hot streams inlet pipe 18, which are therefore correspondingly advantageously cooled, i.e. by those condensations, at about 98°C, which are normally generated by the condensation of the damp steam extracted, at the end of sterilization, from inside the sterilization chamber 52 and/or by possible other steam services of the machine 50. Possibly, other steam condensations can be extracted from the temperature maintenance interspace 55 at the end of the working day.

In this way, in embodiments described using figs. 1, 2, 3 and 4, it is possible to feed the steam generator 54, thanks to the de-mineralized water exit pipe 30, no longer with cold de-mineralized water, as happens in the state of the art, but with already pre-heated de-mineralized water, advantageously at a temperature in the order of 60°C-70°C, for example 65°C, with considerable saving of energy, costs and times. Furthermore, the quantity of de-mineralized water accumulated and pre-heated in the heat exchange chamber 12 forms a safety accumulation or backup of de-mineralized water, for example a few liters (seven, eight, nine, ten or even more than ten accumulated liters), to deal with those moments when the source of de-mineralized water, for various reasons, is momentarily not available, and thus allows in any case to complete the sterilization cycle in progress even if the supply of de-mineralized water is interrupted.

According to some embodiments described here, the functioning of the machine 50 provides that, before the start of each sterilization cycle, an operation to validate the machine 50 is carried out, to verify that the temperature and steam penetration capacity are suitable for sterilization. To this purpose, it is provided to put the sterilization chamber 52 under vacuum once or several times, by means of the suction device 56, for example performing a first vacuum operation to extract air, followed by the introduction of sterilization steam at about 134°C produced by the steam generator 54, and then followed, generally, by two other vacuum operations to extract steam mixed with air. The steam produced by the steam generator 54 is also introduced into the temperature maintenance interspace 55, for the purposes described above.

The streams of air and steam mixed with air extracted from the sterilization chamber 52 are those which, as described above, are made to flow through the vacuum extraction branch of a mixed hot stream 76 to the heat exchanger 14 where they are cooled by the cold water arriving from the cooling water introduction pipe 34 and then, passing through the suction device 56, are introduced and accumulated in the water collection chamber 16 from where, now cooled, they are used, thanks to the condensation water outlet pipe 24, for the functioning of the suction device 56, or will be freely discharged if the level is excessive, by the overflow vent or discharge element 23.

Once the sterilization chamber 52 has been prepared using these vacuum operations, and validation has been positively made, the sterilization cycle can start in the sterilization chamber 52. As described above, during the sterilization, steam is extracted from the sterilization chamber 52 in the form of condensation, i.e. water at about 98°C, which is introduced by means of the hot streams inlet pipe 18 and cooled in the heat exchange chamber 12. For example, in embodiments described using figs. 1, 2, 3 and 4, the condensation introduced by the hot streams inlet pipe 18 is associated with the heat exchange element 20 and is used to pre-heat the de-mineralized water in the heat exchange chamber 12.

At the end of the sterilization cycle, other vacuum cycles are typically carried out, to extract the steam from the sterilization chamber 52 by the suction device 56, and to perform drying, after which the sterilized objects can be extracted. In this case too, by means of the vacuum extraction branch of a mixed hot stream 76, the hot stream is made to pass through the heat exchanger 14 and from here, once cooled, introduced into the suction device 56 which supplies it to the water collection chamber 16, as happens for the steam mixed with air that is vacuum extracted before the start of the sterilization cycle.

Figs. 5 and 6 are used to describe embodiments, combinable with all the embodiments described here, of the apparatus 10 which, compared with the embodiments described using figs. 1-4, essentially does not provide to recover the heat given up in the cooling of the condensations arriving from the hot streams inlet pipe 18 to pre-heat the de-mineralized water supplied to the steam generator 54. In these embodiments, the apparatus 10 only provides to cool the discharges of hot streams and water entering the suction device 56, i.e. condensations arriving from the hot streams inlet pipe 18 and mixed hot streams arriving from the vacuum extraction branch of a mixed hot stream 76. Therefore, in the heat exchange chamber 12 used in the embodiments described with reference to figs. 5 and 6, no de-mineralized water is introduced and the heat exchange chamber 12 itself therefore does not function as an accumulation of pre-heated de-mineralized water. In these embodiments, the de-mineralized water supply pipe 38 is not connected to the heat exchange chamber 12 but is connected directly to the entrance of the de-mineralized water introduction pump 32, which is provided to supply the de-mineralized water to the steam generator 54. Consequently, in these embodiments the de-mineralized water exit pipe 30 is not included, since the de-mineralized water is not pre-heated in the heat exchange chamber 12. In embodiments described using figs. 5 and 6, moreover, the heat exchange chamber 12 and the water collection chamber 16 can be incorporated in a single container 13, as described before, for example providing that the heat exchange chamber 12 is disposed below the water collection chamber 16. This solution in which the heat exchange chamber 12 is disposed below the water collection chamber 16 therefore provides that excess water from the upper water collection chamber 16 is transferred, by means of the transfer pipe 22, into the heat exchange chamber 12 below, where it accumulates and can be mixed with possible water already present and where it is involved in a heat exchange with the condensations arriving from the hot streams inlet pipe 18 and which flow into the heat exchange element 20.

Cold water arriving from the water collection chamber 16 is added to the water present in the heat exchange chamber 12 by means of the transfer pipe 22.

The condensations arriving from the hot streams inlet pipe 18, once they have passed through the heat exchange element 20 and therefore cooled thanks to the heat exchange with the water in the heat exchange chamber 12, are freely discharged into the heat exchange chamber 12 itself.

Once the level of the water reaches a certain threshold due to the contribution of the cooled condensations and the water arriving from the transfer pipe 22, the accumulated water is discharged from the heat exchange chamber 12 through the overflow vent or discharge element 23 of the heat exchange chamber 12. A thermostat device 19 can be provided, which monitors the temperature of the water in the heat exchange chamber 12, so that it remains in a predefined and desired temperature range, for example between 50°C and 60°C, and such as to obtain the necessary cooling of the condensations that flow in the heat exchange element 20. In this case too, moreover, a thermostat device 19 can be provided, associated with the water collection chamber 16, to thermostat and stabilize the accumulated water at about 30°C-40°C, with functions similar to those described above.

Also in the embodiments described with reference to figs. 5 and 6, it is possible to make the suction device 56 function at a lower temperature than in the state of the art. This is because the vacuum outlet pipe for the cooled streams 77, in which the fluid flows arriving from the vacuum extraction branch of a mixed hot stream 76 and cooled by the heat exchange obtained in the heat exchanger 14, is connected at entrance to the suction device 56, with all the advantages described above. Moreover, the suction device 56 can operate at a lower temperature also because it is possible to introduce into it cooling water arriving from the water collection chamber 16 by means of the condensation water outlet pipe 24, in this case too with all the advantages described above.

Advantageously, with the embodiments described here using all the figs. from 1 to 6, thanks to the fact that the cold water that is normally used for the functioning of the suction device 56 is used first of all to cool the air, the steam mixed with air or steam that are vacuum extracted at the start and end of the sterilization cycle, the suction device 56 is no longer subject to accumulations of lime scale and consequent drop in performance and, in general, temperatures too high that would reduce its working life.

Furthermore, since the discharges from the overflow vent or discharge elements 23 of the heat exchange chamber 12 or the water collection chamber 16 consist of water which is no longer at high temperature, it is no longer necessary to provide "ad hoc" drains of the forced type made of steel or cast iron. Moreover, in the case of batteries of sterilization machines, the advantage is obtained of preventing the problem of the backward super-pressure effects due to a single common drain. In this way, the disadvantage that sterilization machines upstream in the battery do not reach the correct temperature because of these super-pressure effects is overcome.

Consequently, it is obvious that the apparatus 10 and machine 50 described here can allow to manage efficiently the heat energy content of hot streams associated with the machine 50, for example supplying the suction device with a stream cooled by the cold water normally used for the functioning of the suction device itself, and producing discharges to the water discharge line that are colder, normally at about 40°C-60°C, and therefore manageable more simply and economically. Therefore, the apparatus 10 and functioning method connected to it allow both an energy recovery of the hot streams, and also an optimization of the temperature of said hot streams, compatible with working conditions of the suction device that are less stressing from the point of view of temperature. Consequently, the machine 50 and sterilization method connected to it are more reliable, since the validation of the required temperature and sterilization conditions is guaranteed.

The apparatus 10 and machine 50 described here are also advantageous because, with reference for example to embodiments described using figs. 1, 2, 3 and 4, they allow to recover the energy content associated with the hot streams associated with the machine 50, in particular the condensations arriving from the hot streams inlet pipe 18, to pre-heat the de-mineralized water to be supplied to the steam generator 54. Furthermore, it is clear that, as well as the advantage of pre-heating the de-mineralized water used to generate the steam by exploiting the hot condensations arriving from the sterilization chamber 52, thanks to the accumulation of pre-heated de-mineralized water in the heat exchange chamber 12, a backup or store of de-mineralized water is created, useful for completing the sterilization cycle in progress, in the event of an interruption from the source of de-mineralized water.

It is clear that modifications and/or additions of parts and/or steps may be made to the apparatus 10 to manage the energy of hot streams of a sterilization machine, the sterilization chamber 50 comprising said apparatus and the corresponding method to manage the energy of hot streams and sterilization method as described heretofore, without departing from the field and scope of the present invention as defined by the claims.

It is also clear that, although the present invention has been described with reference to some specific examples, a person of skill in the art shall certainly be able to achieve many other equivalent forms of apparatus 10 to manage the energy of hot streams of a sterilization machine, the sterilization chamber 50 comprising said apparatus and the corresponding method to manage the energy of hot streams and sterilization method, having the characteristics as set forth in the claims and hence all coming within the field of protection defined thereby.

Although the above refers to embodiments of the invention, other embodiments can be provided without departing from the main field of protection, which is defined by the following claims.

In the following claims, the sole purpose of the references in brackets is to facilitate reading: they must not be considered as restrictive factors with regard to the field of protection claimed in the specific claims.

## Claims

1. Apparatus to manage the energy of hot streams of a sterilization machine (50), comprising :
- a heat exchange chamber (12);
- a heat exchanger (14);
- a water collection chamber (16);
wherein the heat exchange chamber (12) is configured to be at least connected at entrance to an inlet pipe for hot streams (18) arriving from a sterilization chamber (52) of the sterilization machine (50), a heat exchange element (20) being provided inserted into the heat exchange chamber (12) and connected to the hot streams inlet pipe (18),
wherein the heat exchanger (14) is configured to be connected at entrance to a cooling water inlet pipe (34) and to a vacuum extraction branch of a mixed hot stream (76) connected to the sterilization chamber (52) and at exit to a cooling water introduction pipe (28) connected to the water collection chamber (16) and to an outlet pipe for the cooled streams (77),
wherein the water collection chamber (16) is connected to the heat exchange chamber (12) by means of a transfer pipe (22) and is configured to be connected at entrance to said cooling water introduction pipe (28) and wherein said water collection chamber (16) is also connected in use to a suction device (56) at entrance via a vacuum circuit water inlet pipe (26) to supply water from the suction device (56) to the water collection chamber (16) and at exit via a condensation water outlet pipe (24) to supply condensation water from said water collection chamber (16) to said suction device (56).

2. Apparatus as in claim 1, wherein said suction device (56) is connected to said outlet pipe for the cooled streams (77), said suction device (56) is connected at exit to said vacuum circuit water inlet pipe (26) and said suction device (56) also is connected to an entrance of said condensation water outlet pipe (24).

3. Apparatus as in claim 1 or 2, wherein said heat exchange element (20) is a heat exchange coil connected to the hot streams inlet pipe (18).

4. Apparatus as in claim 1, 2 or 3, said apparatus also comprising a level-switch device (21) provided in the heat exchange chamber (12).

5. Apparatus as in any of the claims from 1 to 4, said apparatus also comprising, a thermostat device (19) provided at least in the water collection chamber (16).

6. Apparatus as in any of the claims from 1 to 5, wherein both the heat exchange chamber (12), and the water collection chamber (16) are provided with overflow vent or discharge elements (23).

7. Apparatus as in any of the claims from 1 to 6, wherein the heat exchange chamber (12) is configured to perform a pre-heating of de-mineralized water contained therein by means of hot streams received from the sterilization chamber (52) and is configured to be further connected at entrance to a de-mineralized water supply pipe (38) and at exit, during use, to a steam generator (54) able to use the pre-heated de-mineralized water to generate steam.

8. Apparatus as in claim 7, wherein said apparatus comprises said steam generator (54).

9. Container to manage the energy of hot streams of a sterilization machine (50), comprising:
- a heat exchange chamber (12) and a water collection chamber (16) disposed above or below the heat exchange chamber (12),
- at least one heat exchange element (20) provided in the heat exchange chamber (12);
- a transfer pipe (22) between the heat exchange chamber (12) and the water collection chamber (16);
wherein the heat exchange chamber (12) is provided at least with an entrance, associated with said heat exchange element (20), connecting a hot streams inlet pipe (18) arriving from a sterilization chamber (52) of the sterilization machine (50);
wherein the water collection chamber (16) is provided at least with a connection entrance of a cooling water introduction pipe (28) and is connected in use to a suction device (56) via a connection entrance of a vacuum circuit water inlet pipe (26) to supply water from the suction device (56) to the water collection chamber (16) and via a connection exit of a condensation water outlet pipe (24) to supply condensation water from said water collection chamber (16) to said suction device (56).

10. Container as in claim 9, said container comprising a separation wall (15) between the heat exchange chamber (12) and the water collection chamber (16).

11. Container as in claim 10, wherein said transfer pipe (22) is made through said separation wall (15).

12. Method to manage the energy of hot streams of a sterilization machine (50), comprising:
- cooling, in a heat exchange chamber (12), hot streams arriving from a sterilization chamber (52) of the sterilization machine (50);
- performing a heat exchange, by means of a heat exchanger (14), between cold water and a mixed hot stream arriving from the sterilization chamber (52), to cool the mixed hot stream, wherein the mixed hot stream comprises air and/or steam mixed with air and/or steam;
- introducing the cold water subjected to heat exchange in the heat exchanger (14) into a water collection chamber (16) and introducing the cooled mixed hot stream into a suction device (56);
- transferring into the water collection chamber (16) the cooled exhausted hot streams in the heat exchange chamber (12);
- introducing into the suction device (56) the water contained in the water collection chamber (16), mixing it with the cooled mixed hot stream arriving from the heat exchanger (14);
- transferring into the water collection chamber (16) the mixed hot stream cooled and mixed with the water contained in the water collection chamber (16) at exit from the suction device (56);
- discharging freely into a water discharge line (57) an excess liquid present in the heat exchange chamber (12) and an excess water in the water collection chamber (16).

13. Method as in claim 12, wherein said method provides to pre-heat de-mineralized water contained in the heat exchange chamber (12) to a temperature between 60°C and 70°C.

14. Method as in claim 12 or 13, wherein the suction device (56) comprises a liquid-ring vacuum pump or a Venturi effect pump and wherein the heat exchange in the heat exchanger (14) and the mixing of the water contained in the water collection chamber (16), and a cooled mixed hot stream arriving from the heat exchanger (14) are such that the suction device (56) operates with the water at a temperature between 40°C and 60°C.

15. Method as in claim 12, 13 or 14, wherein the water present in the water collection chamber (16) is kept at a temperature between 40°C and 60°C.

16. Method as in any of the claims from 12 to 15, wherein, in the heat exchange chamber (12), cooling the hot streams arriving from the sterilization chamber (52), a pre-heating of de-mineralized water contained in the heat exchange chamber (12) is performed, said method providing to supply the pre-heated de-mineralized water to a steam generator (54) to produce the steam necessary for the sterilization chamber (52).

17. Machine for sterilizing objects, comprising:
- a sterilization chamber (52),
- a steam generator (54),
- a suction device (56),
- an apparatus (10) to manage the energy of hot streams as in any of the claims from 1 to 8 that comprises:
- a heat exchange chamber (12);
- a heat exchanger (14);
- a water collection chamber (16);
wherein the heat exchange chamber (12) is at least connected at entrance to a hot streams inlet pipe (18) arriving from the sterilization chamber (52),
wherein the heat exchanger (14) is connected at entrance to a cooling water inlet pipe (34) and to a vacuum extraction branch of mixed hot stream (76) connected to the sterilization chamber (52) and at exit to a cooling water introduction pipe (28) connected to the water collection chamber (16) and to an outlet pipe for the cooled streams (77) connected to the suction device (56),
and wherein the water collection chamber (16) is connected to the heat exchange chamber (12) by means of a transfer pipe (22) and is connected at entrance to said cooling water introduction pipe (28) and also to an inlet pipe for the water of the vacuum circuit (26) arriving from an exit of the suction device (56) and at exit to a condensation water outlet pipe (24) connected at entrance to the suction device (56).

18. Machine as in claim 17, wherein the heat exchange chamber (12) is configured to perform a pre-heating of de-mineralized water contained therein and is configured to be further connected at entrance to a de-mineralized water supply pipe (38) and at exit to a steam generator (54) able to use the pre-heated de-mineralized water for the generation of steam.

19. Method for sterilizing objects, comprising:
- putting under vacuum once or several times a sterilization chamber (52) of a sterilization machine (50) to extract a mixed hot stream, by means of a suction device (56);
- producing and introducing steam into the sterilization chamber (52) to perform a sterilization operation, extracting steam from the sterilization chamber (52) that is condensed, producing hot streams;
- drying the sterilized objects, putting under vacuum once or several times the sterilization chamber (52) extracting another mixed hot stream, by means of the suction device (56);
wherein said method also provides an energy management of hot streams in said sterilization machine (50) as in any of the claims from 12 to 16 that comprises:
- cooling in a heat exchange chamber (12), said hot streams arriving from the sterilization chamber (52);
- performing a heat exchange, by means of a heat exchanger (14), between cold water and said mixed hot stream arriving from the sterilization chamber (52), to cool the mixed hot stream;
- introducing the cold water subjected to heat exchange in the heat exchanger (14) into a water collection chamber (16) and introducing the cooled mixed hot stream into a suction device (56);
- transferring into the water collection chamber (16) the cooled exhausted hot streams in the heat exchange chamber (12);
- introducing into the suction device (56) the water contained in the water collection chamber (16), mixing it with the cooled mixed hot stream arriving from the heat exchanger (14);
- transferring into the water collection chamber (16) the mixed hot stream cooled and mixed with the water contained in the water collection chamber (16) at exit from the suction device (56);
- discharging freely into a water discharge line (57) an excess liquid present in the heat exchange chamber (12) and the excess water in the water collection chamber (16).

20. Method as in claim 19, wherein, in the heat exchange chamber (12), cooling the hot streams arriving from the sterilization chamber (52), a pre-heating of the de-mineralized water contained in the heat exchange chamber (12) is performed, said method providing to supply the pre-heated de-mineralized water to a steam generator (54) to produce the steam necessary for the sterilization chamber (52).

## Patentansprüche

1. Gerät zur Verwaltung der Energie von heißen Strömen einer Sterilisationsmaschine (50), umfassend:
- eine Wärmeaustauschkammer (12);
- einen Wärmeaustauscher (14);
- eine Wassersammelkammer (16);
worin die Wärmeaustauschkammer (12) dafür eingerichtet ist, um am Eingang mit einem Eintrittsrohr für heiße Ströme (18) zumindest verbunden zu sein, die aus einer Sterilisationskammer (52) der Sterilisationsmaschine (50) kommen, wobei ein Wärmeaustauschelement (20) vorgesehen ist, das in die Wärmeaustauschkammer (12) eingefügt und mit dem Eintrittsrohr für heiße Ströme (18) verbunden ist,
worin der Wärmeaustauscher (14) dafür eingerichtet ist, um am Eingang mit einem Kühlwassereintrittsrohr (34) und mit einem Vakuumextraktionszweig eines gemischten heißen Stroms (76), der mit der Sterilisationskammer (52) verbunden ist, und am Ausgang mit einem Kühlwassereinführungsrohr (28) verbunden zu sein, das mit der Wassersammelkammer (16) und mit einem Austrittsrohr für die gekühlten Ströme (77) verbunden ist,
worin die Wassersammelkammer (16) mit der Wärmeaustauschkammer (12) über ein Überführungsrohr (22) verbunden ist und dafür eingerichtet ist, um am Eingang mit dem genannten Kühlwassereinführungsrohr (28) verbunden zu sein, und worin die Wassersammelkammer (16) während der Benutzung auch mit einer Saugvorrichtung (56) am Eingang über ein Vakuumkreislaufwassereintrittsrohr (26) verbunden ist, um Wasser aus der Saugvorrichtung (56) der Wassersammelkammer (16) zuzuleiten, und am Ausgang über ein Kondenswasseraustrittsrohr (24), um Kondenswasser aus der Wassersammelkammer (16) der Saugvorrichtung (56) zuzuleiten, verbunden ist.

2. Gerät nach Anspruch 1, worin die Saugvorrichtung (56) mit dem Austrittsrohr für die gekühlten Ströme (77) verbunden ist, die Saugvorrichtung (56) am Ausgang mit dem Vakuumkreislaufwassereintrittsrohr (26) verbunden ist und die Saugvorrichtung (56) auch mit einem Eingang des Kondenswasseraustrittsrohres (24) verbunden ist.

3. Gerät nach Anspruch 1 oder 2, worin das Wärmeaustauschelement (20) eine Wärmeaustauschschlange ist, die mit dem Eintrittsrohr für heiße Ströme (18) verbunden ist.

4. Gerät nach Anspruch 1, 2 oder 3, wobei das Gerät auch eine Niveauschaltvorrichtung (21) umfasst, die in der Wärmeaustauschkammer (12) vorgesehen ist.

5. Gerät nach einem der Ansprüche 1 bis 4, wobei das Gerät auch eine Thermostatvorrichtung (19) umfasst, die zumindest in der Wassersammelkammer (16) vorgesehen ist.

6. Gerät nach einem der Ansprüche 1 bis 5, worin sowohl die Wärmeaustauschkammer (12) und die Wassersammelkammer (16) mit Überlauföffnungs- oder Ablasselementen (23) versehen sind.

7. Gerät nach einem der Ansprüche 1 bis 6, worin die Wärmeaustauschkammer (12) dafür eingerichtet ist, um ein Vorwärmen von darin enthaltenem entmineralisiertem Wasser mittels heißer Ströme durchzuführen, die von der Sterilisationskammer (52) empfangen werden, und dafür eingerichtet ist, um am Eingang ferner mit einem Zuführungsrohr (38) von entmineralisiertem Wasser und am Ausgang, während der Benutzung, mit einem Dampferzeuger (54) verbunden zu werden, der imstande ist, das vorgewärmte entmineralisierte Wasser zu verwenden, um Dampf zu erzeugen.

8. Gerät nach Anspruch 7, worin das Gerät den genannten Dampferzeuger (54) umfasst.

9. Behälter zur Verwaltung der Energie von heißen Strömen einer Sterilisationsmaschine (50), umfassend:
- eine Wärmeaustauschkammer (12) und eine Wassersammelkammer (16), die über oder unten der Wärmeaustauschkammer (12) angeordnet ist;
- zumindest ein Wärmeaustauschelement (20), das in der Wärmeaustauschkammer (12) vorgesehen ist;
- ein Überführungsrohr (22) zwischen der Wärmeaustauschkammer (12) und der Wassersammelkammer (16); worin die Wärmeaustauschkammer (12) zumindest mit einem Eingang versehen ist, das dem Wärmeaustauschelement (20) zugeordnet ist, das ein Eintrittsrohr für heiße Ströme (18), die aus einer Sterilisationskammer (52) der Sterilisationsmaschine (50) kommen, verbindet;
worin die Wassersammelkammer (16) zumindest mit einem Verbindungseingang eines Kühlwassereinführungsrohres (28) versehen ist und während der Benutzung mit einer Saugvorrichtung (56) über einen Verbindungseingang eines Vakuumkreislaufwassereintrittsrohres (26) verbunden ist, um Wasser aus der Saugvorrichtung (56) der Wassersammelkammer (16) zuzuleiten, und über einen Verbindungsausgang eines Kondenswasseraustrittsrohres (24) verbunden ist, um Kondenswasser aus der Wassersammelkammer (16) der Saugvorrichtung (56) zuzuleiten.

10. Behälter nach Anspruch 9, wobei der Behälter eine Trennwand (15) zwischen der Wärmeaustauschkammer (12) und der Wassersammelkammer (16) umfasst.

11. Behälter nach Anspruch 10, wobei das Überführungsrohr (22) durch die Trennwand (15) ausgebildet ist.

12. Verfahren zur Verwaltung der Energie von heißen Strömen einer Sterilisationsmaschine (50), umfassend:
- Kühlen, in einer Wärmeaustauschkammer (12), von heißen Strömen, die aus einer Sterilisationskammer (52) der Sterilisationsmaschine (50) kommen;
- Durchführen eines Wärmeaustausches, mittels eines Wärmeaustauschers (14), zwischen kaltem Wasser und einem gemischten heißen Strom, der aus der Sterilisationskammer (52) kommt, um den gemischten heißen Strom zu kühlen, worin der gemischte heiße Strom Luft und/oder mit Luft gemischten Dampf und/oder Dampf umfasst;
- Einführen des kalten Wassers, das dem Wärmeaustausch im Wärmeaustauscher (14) unterzogen wird, in eine Wassersammelkammer (16) hinein und Einführen des gekühlten gemischten heißen Stroms in eine Saugvorrichtung (56) hinein;
- Überführen in die Wassersammelkammer (16) hinein der gekühlten erschöpften heißen Ströme in der Wärmeaustauschkammer (12);
- Einführen in die Saugvorrichtung (56) hinein des Wassers, das in der Wassersammelkammer (16) enthalten ist, Mischen desselben mit dem gekühlten gemischten heißen Stroms, der aus dem Wärmeaustauscher (14) kommt;
- Überführen in die Wassersammelkammer (16) hinein des gemischten heißen Stroms, der gekühlt und mit dem Wasser gemischt wird, das in der Wassersammelkammer (16) am Ausgang aus der Saugvorrichtung (56) enthalten ist;
- Abführen in freier Weise in eine Wasserabfuhrleitung (57) einer überschüssigen Flüssigkeit, die in der Wärmeaustauschkammer (12) vorhanden ist, und eines überschüssigen Wassers in der Wassersammelkammer (16).

13. Verfahren nach Anspruch 12, worin das Verfahren vorsieht, entmineralisiertes Wasser, das in der Wärmeaustauschkammer (12) enthalten ist, auf eine Temperatur zwischen 60°C und 70°C vorzuwärmen.

14. Verfahren nach Anspruch 12 oder 13, worin die Saugvorrichtung (56) eine Flüssigkeitsringvakuumpumpe oder eine Venturi-Effekt-Pumpe umfasst und worin der Wärmeaustausch in dem Wärmeaustauscher (14) und das Mischen des Wassers, das in der Wassersammelkammer (16) enthalten ist, und ein gekühlter gemischter heißer Strom, der aus dem Wärmeaustauscher (14) kommt, solche sind, dass die Saugvorrichtung (56) mit dem Wasser bei einer Temperatur zwischen 40°C und 60°C arbeitet.

15. Verfahren nach Anspruch 12, 13 oder 14, worin das Wasser, das in der Wassersammelkammer (16) vorhanden ist, bei einer Temperatur zwischen 40°C und 60°C gehalten wird.

16. Verfahren nach einem der Ansprüche 12 bis 15, worin, in der Wärmeaustauschkammer (12), durch Kühlen der heißen Ströme, die aus der Sterilisationskammer (52) kommen, ein Vorwärmen von entmineralisiertem Wasser, das in der Wärmeaustauschkammer (12) enthalten ist, durchgeführt wird, wobei das Verfahren vorsieht, das vorgewärmte entmineralisierte Wasser einem Dampferzeuger (54) zuzuleiten, um den Dampf zu erzeugen, der für die Sterilisationskammer (52) erforderlich ist.

17. Maschine zur Sterilisation von Gegenständen, umfassend:
- eine Sterilisationskammer (52),
- einen Dampferzeuger (54),
- eine Saugvorrichtung (56),
- ein Gerät (10) zur Verwaltung der Energie von heißen Strömen nach einem der Ansprüche 1 bis 8, das umfasst:
- eine Wärmeaustauschkammer (12);
- einen Wärmeaustauscher (14);
- eine Wassersammelkammer (16);
worin die Wärmeaustauschkammer (12) am Eingang mit einem Eintrittsrohr für heiße Ströme (18) zumindest verbunden ist, die aus der Sterilisationskammer (52) kommen,
worin der Wärmeaustauscher (14) am Eingang mit einem Kühlwassereintrittsrohr (34) und mit einem Vakuumextraktionszweig von gemischtem heißem Strom (76), der mit der Sterilisationskammer (52) verbunden ist, und am Ausgang mit einem Kühlwassereinführungsrohr (28) verbunden ist, das mit der Wassersammelkammer (16) und mit einem Austrittsrohr für die gekühlten Ströme (77) verbunden ist, das mit der Saugvorrichtung (56) verbunden ist,
und worin die Wassersammelkammer (16) mit der Wärmeaustauschkammer (12) über ein Überführungsrohr (22) verbunden ist und am Eingang mit dem genannten Kühlwassereinführungsrohr (28) verbunden ist, und auch mit einem Eintrittsrohr für das Wasser des Vakuumkreislaufs (26), das aus einem Ausgang der Saugvorrichtung (56) kommt, und am Ausgang mit einem Kondenswasseraustrittsrohr (24) verbunden ist, das am Eingang mit der Saugvorrichtung (56) verbunden ist.

18. Maschine nach Anspruch 17, worin die Wärmeaustauschkammer (12) dafür eingerichtet ist, um ein Vorwärmen von darin enthaltenem entmineralisiertem Wasser durchzuführen, und dafür eingerichtet ist, um am Eingang ferner mit einem Zuführungsrohr (38) von entmineralisiertem Wasser und am Ausgang mit einem Dampferzeuger (54) verbunden zu werden, der imstande ist, das vorgewärmte entmineralisierte Wasser zur Dampferzeugung zu verwenden.

19. Verfahren zur Sterilisation von Gegenständen, umfassend:
- Unter-Vakuum-Setzen einmal oder mehrere Male einer Sterilisationskammer (52) einer Sterilisationsmaschine (50), um einen gemischten heißen Strom, mittels einer Saugvorrichtung (56), unter Vakuum zu extrahieren;
- Erzeugen und Einführen von Dampf in die Sterilisationskammer (52), um einen Sterilisationsvorgang durchzuführen, indem Dampf aus der Sterilisationskammer (52) extrahiert wird, der kondensiert wird, wodurch heiße Ströme erzeugt werden;
- Trocknen der sterilisierten Gegenstände, durch Unter-Vakuum-Setzen einmal oder mehrere Male der Sterilisationskammer (52), indem ein weiterer gemischter heißer Strom, mittels der Saugvorrichtung (56), extrahiert wird;
worin das Verfahren auch eine Verwaltung der Energie von heißen Strömen in der Sterilisationsmaschine (50) nach einem der Ansprüche 12 bis 16 vorsieht, die umfasst:
- Kühlen in einer Wärmeaustauschkammer (12) der genannten heißen Ströme, die aus der Sterilisationskammer (52) kommen;
- Durchführen eines Wärmeaustausches, mittels eines Wärmeaustauschers (14), zwischen kaltem Wasser und dem gemischten heißen Strom, der aus der Sterilisationskammer (52) kommt, um den gemischten heißen Strom zu kühlen;
- Einführen des kalten Wassers, das dem Wärmeaustausch im Wärmeaustauscher (14) unterzogen wird, in eine Wassersammelkammer (16) hinein und Einführen des gekühlten gemischten heißen Stroms in eine Saugvorrichtung (56) hinein;
- Überführen in die Wassersammelkammer (16) hinein der gekühlten erschöpften heißen Ströme in der Wärmeaustauschkammer (12);
- Einführen in die Saugvorrichtung (56) hinein des Wassers, das in der Wassersammelkammer (16) enthalten ist, Mischen desselben mit dem gekühlten gemischten heißen Stroms, der aus dem Wärmeaustauscher (14) kommt;
- Überführen in die Wassersammelkammer (16) hinein des gemischten heißen Stroms, der gekühlt und mit dem Wasser gemischt wird, das in der Wassersammelkammer (16) am Ausgang aus der Saugvorrichtung (56) enthalten ist;
- Abführen in freier Weise in eine Wasserabfuhrleitung (57) einer überschüssigen Flüssigkeit, die in der Wärmeaustauschkammer (12) vorhanden ist, und des überschüssigen Wassers in der Wassersammelkammer (16).

20. Verfahren nach Anspruch 19, worin, in der Wärmeaustauschkammer (12), durch Kühlen der heißen Ströme, die aus der Sterilisationskammer (52) kommen, ein Vorwärmen des entmineralisierten Wassers, das in der Wärmeaustauschkammer (12) enthalten ist, durchgeführt wird, wobei das Verfahren vorsieht, das vorgewärmte entmineralisierte Wasser einem Dampferzeuger (54) zuzuleiten, um den Dampf zu erzeugen, der für die Sterilisationskammer (52) erforderlich ist.

## Revendications

1. Appareil pour gérer l'énergie de flux chauds d'une machine de stérilisation (50), comprenant :
- une chambre d'échange de chaleur (12) ;
- un échangeur de chaleur (14) ;
- une chambre de collecte d'eau (16) ;
dans lequel la chambre d'échange de chaleur (12) est configurée pour être au moins connectée au niveau de l'entrée à un tuyau d'entrée pour des flux chauds (18) provenant d'une chambre de stérilisation (52) de la machine de stérilisation (50), un élément d'échange de chaleur (20) étant prévu inséré dans la chambre d'échange de chaleur (12) et connecté au tuyau d'entrée de flux chauds (18),
dans lequel l'échangeur de chaleur (14) est configuré pour être connecté au niveau de l'entrée à un tuyau d'entrée d'eau de refroidissement (34) et à une branche d'extraction sous vide d'un flux chaud mixte (76) connecté à la chambre de stérilisation (52) et au niveau de la sortie à un tuyau d'introduction d'eau de refroidissement (28) connecté à la chambre de collecte d'eau (16) et à un tuyau de sortie des courants refroidis (77),
dans lequel la chambre de collecte d'eau (16) est connectée à la chambre d'échange de chaleur (12) au moyen d'un tuyau de transfert (22) et est configurée pour être connectée au niveau de l'entrée audit tuyau d'introduction d'eau de refroidissement (28) et dans lequel ladite chambre de collecte d'eau (16) est également connectée en utilisation à un dispositif d'aspiration (56) au niveau de l'entrée via un tuyau d'entrée d'eau de circuit sous vide (26) pour amener l'eau à partir du dispositif d'aspiration (56) jusqu'à la chambre de collecte d'eau (16) et au niveau de la sortie via un tuyau de sortie d'eau de condensation (24) pour fournir de l'eau de condensation à partir de ladite chambre de collecte d'eau (16) jusqu'audit dispositif d'aspiration (56).

2. Appareil selon la revendication 1, dans lequel ledit dispositif d'aspiration (56) est connecté audit tuyau de sortie pour les flux refroidis (77), ledit dispositif d'aspiration (56) est connecté au niveau de la sortie audit tuyau d'entrée d'eau de circuit sous vide (26) et ledit dispositif d'aspiration (56) est également connecté au niveau d'une entrée dudit tuyau de sortie d'eau de condensation (24).

3. Appareil selon la revendication 1 ou 2, dans lequel ledit élément d'échange de chaleur (20) est une bobine d'échange de chaleur connectée au tuyau d'entrée de flux chauds (18).

4. Appareil selon la revendication 1, 2 ou 3, ledit appareil comprenant également un dispositif de commutation de niveau (21) prévu dans la chambre d'échange de chaleur (12).

5. Appareil selon l'une quelconque des revendications 1 à 4, ledit appareil comprenant également un dispositif de thermostat (19) prévu au moins dans la chambre de collecte d'eau (16).

6. Appareil selon l'une quelconque des revendications 1 à 5, dans lequel à la fois la chambre d'échange de chaleur (12) et la chambre de collecte d'eau (16) sont munies d'un trop-plein ou d'éléments de décharge (23).

7. Appareil selon l'une quelconque des revendications 1 à 6, dans lequel la chambre d'échange de chaleur (12) est configurée pour effectuer un préchauffage d'eau déminéralisée contenue à l'intérieur au moyen de flux chauds reçus à partir de la chambre de stérilisation (52), et est configuré pour être en outre connecté au niveau de l'entrée à un tuyau d'alimentation d'eau minéralisée (38) et au niveau de la sortie, en utilisation, à un générateur de vapeur (54) capable d'utiliser l'eau déminéralisée préchauffée pour générer de la vapeur.

8. Appareil selon la revendication 7, dans lequel ledit appareil comprend ledit générateur de vapeur (54).

9. Conteneur pour gérer l'énergie de flux chauds d'une machine de stérilisation (50), comprenant :
- une chambre d'échange de chaleur (12) et une chambre de collecte d'eau (16) disposée au-dessus ou au-dessous de la chambre d'échange de chaleur (12),
- au moins un élément d'échange de chaleur (20) prévu dans la chambre d'échange de chaleur (12) ;
- un tuyau de transfert (22) entre la chambre d'échange de chaleur (12) et la chambre de collecte d'eau (16) ;
dans lequel la chambre d'échange de chaleur (12) est pourvue au moins d'une entrée, associée audit élément d'échange de chaleur (20), se connectant à un tuyau d'entrée de flux chauds (18) provenant d'une chambre de stérilisation (52) de la machine de stérilisation (50) ;
dans lequel la chambre de collecte d'eau (16) est pourvue au moins d'une entrée de connexion d'un tuyau d'introduction d'eau de refroidissement (28) et est connectée en utilisation à un dispositif d'aspiration (56) via une entrée de connexion d'un tuyau d'entrée d'eau de circuit sous vide (26) pour amener l'eau à partir du dispositif d'aspiration (56) jusqu'à la chambre de collecte d'eau (16) et via une sortie de connexion d'un tuyau de sortie d'eau de condensation (24) pour alimenter de l'eau de condensation à partir de ladite chambre de collecte d'eau (16) jusqu'audit dispositif d'aspiration (56).

10. Conteneur selon la revendication 9, ledit conteneur comprenant une paroi de séparation (15) entre la chambre d'échange de chaleur (12) et la chambre de collecte d'eau (16).

11. Récipient selon la revendication 10, dans lequel ledit tube de transfert (22) est réalisé à travers ladite paroi de séparation (15).

12. Méthode pour gérer l'énergie de flux chauds d'une machine de stérilisation (50), comprenant les étapes consistant à :
- refroidir, dans une chambre d'échange de chaleur (12), des flux chauds provenant d'une chambre de stérilisation (52) de la machine de stérilisation (50) ;
- effectuer un échange de chaleur, au moyen d'un échangeur de chaleur (14), entre l'eau froide et un flux chaud mixte provenant de la chambre de stérilisation (52), pour refroidir le courant chaud mixte, dans laquelle le flux chaud mixte comprend de l'air et/ou de la vapeur mélangé avec de l'air et/ou de la vapeur ;
- introduire l'eau froide soumise à un échange de chaleur dans l'échangeur de chaleur (14) jusque dans une chambre de collecte d'eau (16), et introduire le flux chaud mixte refroidi dans un dispositif d'aspiration (56) ;
- transférer jusque dans la chambre de collecte d'eau (16) les flux chauds évacués refroidis dans la chambre d'échange de chaleur (12) ;
- introduire dans le dispositif d'aspiration (56) l'eau contenue dans la chambre de collecte d'eau (16), en la mélangeant avec le flux chaud mixte refroidi provenant de l'échangeur de chaleur (14) ;
- transférer dans la chambre de collecte d'eau (16) le flux chaud mixte refroidi et mélangé avec l'eau contenue dans la chambre de collecte d'eau (16) à la sortie du dispositif d'aspiration (56) ;
- décharger librement dans une conduite d'évacuation d'eau (57) un excès de liquide présent dans la chambre d'échange de chaleur (12) et un excès d'eau dans la chambre de collecte d'eau (16).

13. Méthode selon la revendication 12, dans laquelle ladite méthode permet de préchauffer de l'eau déminéralisée contenue dans la chambre d'échange de chaleur (12) à une température comprise entre 60°C et 70°C.

14. Méthode selon la revendication 12 ou 13, dans laquelle le dispositif d'aspiration (56) comprend une pompe vide à anneau liquide ou une pompe à effet Venturi et dans laquelle l'échange de chaleur dans l'échangeur de chaleur (14) et le mélange de l'eau contenue dans la chambre de collecte d'eau (16), et un flux chaud mixte refroidi provenant de l'échangeur de chaleur (14) sont tels que le dispositif d'aspiration (56) fonctionne avec l'eau à une température comprise entre 40°C et 60°C.

15. Méthode selon la revendication 12, 13 ou 14, dans laquelle l'eau présente dans la chambre de collecte d'eau (16) est maintenue à une température comprise entre 40°C et 60°C.

16. Méthode selon l'une quelconque des revendications 12 à 15, dans laquelle, dans la chambre d'échange de chaleur (12), pour un refroidissement des flux chauds provenant de la chambre de stérilisation (52), un préchauffage d'eau déminéralisée contenue dans la chambre d'échange de chaleur (12) est réalisé, ladite méthode étant prévue pour amener l'eau déminéralisée préchauffée jusqu'à un générateur de vapeur (54) pour produire la vapeur nécessaire à la chambre de stérilisation (52).

17. Machine pour stériliser des objets, comprenant :
- une chambre de stérilisation (52),
- un générateur de vapeur (54),
- un dispositif d'aspiration (56),
- un appareil (10) pour gérer l'énergie de flux chauds selon l'une quelconque des revendications 1 à 8 qui comprend :
- une chambre d'échange de chaleur (12) ;
- un échangeur de chaleur (14) ;
- une chambre de collecte d'eau (16) ;
dans laquelle la chambre d'échange de chaleur (12) est au moins connectée au niveau de l'entrée à un tuyau d'entrée de flux chauds (18) provenant de la chambre de stérilisation (52),
dans laquelle l'échangeur de chaleur (14) est connecté au niveau de l'entrée à un tuyau d'entrée d'eau de refroidissement (34) et à une branche d'extraction sous vide de flux chaud mixte (76) connectée à la chambre de stérilisation (52) et au niveau de la sortie à un tuyau d'introduction d'eau de refroidissement (28) connecté à la chambre de collecte d'eau (16) et à un tuyau de sortie pour les flux refroidis (77) connectés au dispositif d'aspiration (56),
et dans laquelle la chambre de collecte d'eau (16) est connectée à la chambre d'échange de chaleur (12) au moyen d'un tuyau de transfert (22) et est connectée au niveau de l'entrée audit tuyau d'introduction d'eau de refroidissement (28) et également à un tuyau d'entrée pour l'eau du circuit de vide (26) arrivant depuis une sortie du dispositif d'aspiration (56), et au niveau de la sortie à un tuyau de sortie d'eau de condensation (24) connecté au niveau de l'entrée au dispositif d'aspiration (56).

18. Machine selon la revendication 17,
dans laquelle la chambre d'échange de chaleur (12) est configurée pour effectuer un préchauffage d'eau déminéralisée contenue dans celle-ci et est configurée pour être connectée en outre au niveau de l'entrée à un tuyau d'alimentation en eau minéralisée (38) et au niveau de la sortie à un générateur de vapeur (54) capable d'utiliser l'eau déminéralisée préchauffée pour la production de vapeur.

19. Méthode de stérilisation d'objets comprenant les étapes consistant à :
- mettre sous vide une ou plusieurs fois une chambre de stérilisation (52) d'une machine de stérilisation (50) pour extraire un flux chaud mixte, au moyen d'un dispositif d'aspiration (56) ;
- produire et introduire de la vapeur dans la chambre de stérilisation (52) pour effectuer une opération de stérilisation, extraire la vapeur de la chambre de stérilisation (52) qui est condensée, produire des flux chauds ;
- sécher les objets stérilisés, mettre sous vide une ou plusieurs fois la chambre de stérilisation (52), extraire un autre flux chaud mixte, au moyen du dispositif d'aspiration (56) ;
dans laquelle ladite méthode fournit également une gestion d'énergie de flux chauds dans ladite machine de stérilisation (50) selon l'une quelconque des revendications 12 à 16, qui comprend les étapes consistant à :
- refroidir dans une chambre d'échange de chaleur (12) lesdits flux chauds provenant de la chambre de stérilisation (52) ;
- effectuer un échange de chaleur, au moyen d'un échangeur de chaleur (14), entre de l'eau froide et ledit flux chaud mixte provenant de la chambre de stérilisation (52), pour refroidir le flux chaud mixte ;
- introduire l'eau froide soumise à un échange de chaleur dans l'échangeur de chaleur (14) jusque dans une chambre de collecte d'eau (16), et introduire le flux chaud mixte refroidi dans un dispositif d'aspiration (56) ;
- transférer dans la chambre de collecte d'eau (16) les flux chauds évacués refroidis dans la chambre d'échange de chaleur (12) ;
- introduire dans le dispositif d'aspiration (56) l'eau contenue dans la chambre de collecte d'eau (16), la mélanger avec le flux chaud mixte refroidi provenant de l'échangeur de chaleur (14) ;
- transférer dans la chambre de collecte d'eau (16) le flux chaud mixte refroidi et mélangé avec l'eau contenue dans la chambre de collecte d'eau (16) à la sortie du dispositif d'aspiration (56) ;
- décharger librement, dans une conduite d'évacuation d'eau (57), un excès de liquide présent dans la chambre d'échange de chaleur (12) et l'excès d'eau dans la chambre de collecte d'eau (16).

20. Méthode selon la revendication 19, dans laquelle, dans la chambre d'échange de chaleur (12), pour un refroidissement des flux chauds provenant de la chambre de stérilisation (52), un préchauffage de l'eau déminéralisée contenue dans la chambre d'échange de chaleur (12) est réalisé, ladite méthode permettant d'amener l'eau déminéralisée préchauffée jusqu'à un générateur de vapeur (54) pour produire la vapeur nécessaire à la chambre de stérilisation (52).
